(19) **Europäisches Patentamt**
**European Patent Office**
**Office européen des brevets**

(11) **EP 0 996 418 B2**

(12) **NEW EUROPEAN PATENT SPECIFICATION**
After opposition procedure

(45) Date of publication and mention
of the opposition decision:
**08.04.2009 Bulletin 2009/15**

(45) Mention of the grant of the patent:
**12.05.2004 Bulletin 2004/20**

(21) Application number: **98950004.6**

(22) Date of filing: **28.08.1998**

(51) Int Cl.:
***A61K 8/18*** (2006.01)

(86) International application number:
**PCT/EP1998/005704**

(87) International publication number:
**WO 1999/011237 (11.03.1999 Gazette 1999/10)**

(54) **DERMATOLOGICAL COMPOSITIONS**

DERMATOLOGISCHE ZUBEREITUNGEN

COMPOSITION DERMATOLOGIQUE

(84) Designated Contracting States:
**AT BE CH CY DE DK ES FI FR GB GR IE IT LI LU
MC NL PT SE**
Designated Extension States:
**LT LV RO SI**

(30) Priority: **01.09.1997 FR 9710866**

(43) Date of publication of application:
**03.05.2000 Bulletin 2000/18**

(73) Proprietor: **Reckitt & Colman (Overseas) Limited
Slough
Berkshire SL1 3UH (GB)**

(72) Inventors:
• **FRUCTUS, Alain, E.
F-92400 Courbevoie (FR)**
• **BOBIER-RIVAL, Carine
F-95000 Cergy (FR)**

(74) Representative: **Cawdell, Karen Teresa et al
Reckitt Benckiser
Corporate Services Limited
Legal Department - Patents Group
Dansom Lane
Hull
HU8 7DS (GB)**

(56) References cited:
**EP-A- 0 747 047         WO-A-97/05856
WO-A-98/03152         DE-A- 4 140 474
DE-C1- 4 420 625         FR-A- 2 682 296
FR-A- 2 729 050         US-A- 5 621 012**

• **"Physical methods of preservation" STN
INTERNATIONAL, KARLSRUHE. FILE PROMPT,
AN=97:279965, XP002064131**

## EP 0 996 418 B2

**Description**

The present invention relates to a dermatological composition containing no chemical preservative, no perfume and no colouring agent and allowing the appearance of the symptoms of hypersensitivity and/or cutaneous intolerance to be avoided at the time of its topical application.

Sensitive or reactive skins are skins which easily react to allergens or irritants following disorders of cutaneous permeability linked to the alteration of the barrier function of the stratum corneum and to an imbalance in the production of epidermic cytokines.

The modification of the cutaneous permeability gives rise to the appearance of subjective and objective signs.

The appearance of subjective signs is particularly observed when ordinary cosmetic or hygiene products are used. They are defined by itching, twinging, pricking, glowing, stinging and burning sensations.

The objective signs are shown in an irregular fashion by xerosis, by seborrheic dermatitis, by telangiectasia, by squames, by flushing, by vesicles or even by an edema.

According to specialists the subjective and objective signs can appear for a short time immediately after application of the cosmetic product or can be indicated in a transitory fashion or can persist for a longer period of time; all day or in an intermittent fashion during the day. Thus, these signs can be discreet or severe and can require a medical opinion.

Persons who show the symptoms of hypersensitivity and cutaneous intolerance can be:

- carriers of dermatosis (atopy, seborrheic dermatitis)
- subjects who have been subjected to traumatisms (sunburn, burns)
- subjects with no previous dermatological history but in whom the subjective and objective signs are present
- subjects who have previously had medicinal treatments.

Thus, it has recently been shown that, except for cosmetic or hygiene products used daily, several factors linked to age, sex, lifestyle and the profession of patients as well as the environment, appear to intervene in cutaneous sensitisation or irritation.

Among these factors, the age of the patient is a significant factor. In fact, infants show a more significant cutaneous sensitivity than adults. Women have more significant hypersensitivity problems and cutaneous intolerance than men. Hormonal cyclic variations greatly influence the reactivity of a woman's skin. In the same way emotions, the stress of daily life, the consumption of alcohol and spicy food, the use of water and detergents during the day, variations in temperature, pollution, also appear to influence the appearance of cutaneous sensitivity.

However, specialists still question the short-term and long-term consequences of the phenomena of hypersensitivity and cutaneous intolerance. Recent studies have shown that no link exists between intolerant skin and the presence of a pre-existing illness. On the contrary, certain studies lead one to believe that the fact of having a sensitive or reactive skin is a factor which encourages a contact allergy. In addition, the in-vivo evaluation of the phenomena of cutaneous irritations by measuring the imperceptible loss of water from the epidermis, has shown that a cutaneous irritation can lead to an interruption of the hydrolipidic film of the surface thus causing an increase in the epidermic loss of water and therefore cutaneous ageing.

It is known from the prior art that certain components of cosmetic or hygiene products, such as chemical preservatives, perfumes, colouring agents, chemical sun screens and ethanol, can cause problems of irritation and/or cutaneous intolerance, even contact allergy problems.

Therefore, the Applicant has sought a solution to the problems of hypersensitivity and cutaneous intolerance by producing a dermatological or cosmetic composition containing no active or raw ingredient of preservative type which could be a potential irritant or allergenic agent.

Dermatological or cosmetic products for non-sterile topical application are exposed to the risks of microbiological contamination during the manufacture, during their packaging and above all during their use by the patient or consumer. For this reason, it is common in the prior art:

- to introduce chemical preservatives, such as parabens and formol donors,
- to introduce natural preservatives, such as essential oil extracts,
- to formulate with a minimum quantity of water,
- to change certain physical factors in the formulation, such as pH, activity of water ($a_w$) and redox potential,
- to incorporate cationic surfactants which have anti-microbial properties,
- to use biological systems such as certain proteins and/or enzymes,
- to use ethanol

in order to better preserve the products.

However, the chemical and/or natural preservatives and the cationic surfactants, despite their anti-microbial power, often cause intolerance reactions, such as irritations and/or allergies.

Moreover, the development of cosmetic products either under conditions of extreme pH or by reducing the quantity of water in the formulation and/or the activity of the water ($a_w$) by an increased quantity of sugar and salt in order to prevent microbial proliferation, no longer satisfies the requirements of scientists and consumers.

The use of certain thickening agents has been proposed such as polyacrylates and polymethacrylates and their derivatives (cf. Patents FR 2 682 296 and WO 97/05856) as anti-microbial compounds in cosmetic compositions. The latter interfere with the activity of water ($a_w$) and exercise a strong osmotic effect on the environment, which allows the inactivation of micro-organisms introduced in a cosmetic or pharmaceutical preparation by depriving them of water. However, their use remains limited, mainly because of the relatively high concentrations which are necessary to obtain the desired anti-microbial effect. At these concentrations, the composition obtained cannot be handled in an efficient manner by the consumer as it is difficult to spread and its consistency is too sticky. In addition, its poor aesthetic appearance does not encourage the consumer to use it again.

The use of hygroscopic agents has also been proposed such as polyols of glycerine, propylene glycol or PEG type as anti-microbial agents. However, their use also remains limited as the high concentrations of this type of compound required to obtain an effective anti-microbial action encourage the active ingredients of the composition to penetrate too deep. The latter then enter into the systemic circulation of the individual and can cause undesirable side effects will seeing their initial topical properties poorly exploited or reduced.

The use of monoalkyl glyceryl ethers in antimicrobial compositions is known. (See EP-A-0747047, FR-A-27229050, DE-A-4140474 and US-A-5621012)

A principal subject of the present invention is a dermatological or cosmetic composition containing no chemical preservative, no perfume and colouring agent and allowing the appearance of the symptoms of hypersensitivity and/or cutaneous intolerance to be avoided. The composition is characterized in that it contains a synergistic mixture of two inhibitors of microbial proliferation.

The inventors have discovered that the simultaneous presence of these two inhibitors of microbial proliferation allows, thanks to a synergistic effect, the mechanism of which is not yet completely elucidated, the substantial reduction in their concentrations. These reduced concentrations allow the desired microbial effect to be obtained without the disadvantages observed during the individual use of these inhibitors.

The present invention therefore provides a dermatological and/or cosmetic composition as claimed in claim 1.

The invention also relates to a dermatological and cosmetic composition which may contain, in addition, at least one polysaccharide gum, such as modified cellulose and/or at least one polysaccharide gum obtained by fermentation such as xanthan gum and/or sclerotium gum, preferably a mixture of these two last-named products.

The cosmetic or dermatological compositions for topical application are often formulated in the form of emulsions. An emulsion is formed by the dispersion of two non-miscible liquids using a surfactant. The emulsions are thermodynamically unstable systems and the stability of an emulsion can be improved by increasing the viscosity of the dispersed and dispersing phases. Moreover, it is known from the prior art that certain gelling agents can stabilise an emulsion without using a surfactant (cf. Patent WO 96/37180).

Another embodiment is the additional use of immunomodulating molecules in the claimed composition, preferably a synthetic peptide to modulate the secretion of inflammation mediators and cytokines as the result of cutaneous irritations.

Allergens are substances which are recognised as "foreign" by the defence system of the skin and induce a contact hypersensitivity or allergy. They are part of our environment. They are present in the air such as pollens, animal hairs, in the water such as calcium salts, in numerous common cosmetic and hygiene products such as perfumes, chemical preservatives, colouring agents and chemical sun screens.

During an allergic or hypersensitivity reaction certain elements of the immune system react violently against the allergens. Therefore, the introduction of an allergen into the skin triggers an acute reaction which manifests itself by an edema, by flushing and by symptoms which manifest themselves later in a prolonged fashion. The inflammation is expressed by an increase in vascular permeability, an orientated afflux of immunocompetent cells (such as lymphoctes, monocytes, macrophages) and the production of inflammation modulators (such as histamine) of cytokines (such as lymphokines, monokines interleukins and interferons) and neurohormones (such as propiomelanocortine, melanotropin, adrenocorticotrope).

All the epidermic cells: keratinocytes, Langerhans' cells, melanocytes and Merkel's cells can secrete inflammation mediators, cytokines and certain neurohormones when faced with a cutaneous irritation. Under normal conditions, the production of cytokines by the epidermic cells is low, however, it is increased during any attack on the epidermis. This hypersecretion and colonisation of tissues by immunocompetent cells are harmful to the organism and need to be modulated as they can damage the tissues.

Therefore, the invention also relates to a dermatological and cosmetic composition as described above and which contains, in addition, an immunoregulating molecule, preferably a synthetic peptide.

Another subject of the present invention is the use of this dermatological and cosmetic composition as described above for the manufacture of or medicament for the treatment of the symptoms of psoriasis.

Another subject of the present invention is the use of this dermatological and cosmetic composition as described above for the manufacture of or medicament for the treatment of the symptoms of atopy.

Another subject of the present invention is the use of this dermatological and cosmetic composition as described above for the manufacture of or medicament for the treatment of the symptoms of acne.

Another subject of the present invention is the use of this dermatological and cosmetic composition as described above for the manufacture of a medicament for the treatment of the symptoms of cutaneous ageing.

Another subject of the present invention is the non-therapeutic use of this dermatological and cosmetic composition as described above for the preparation of sun creams and in particular for the prevention of benign estival light eruption.

This invention as described above is more particularly intended for cosmetic or dermatological non-therapeutic use. More particularly, this dermatological or cosmetic composition according to the invention is applied by topical route as claimed in claim 15.

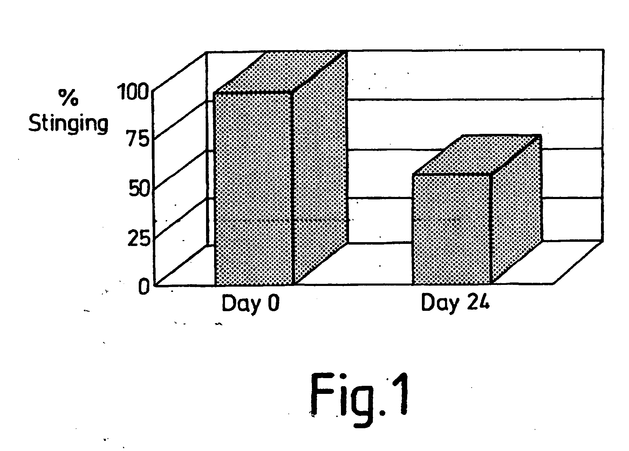

The present invention will now be described in a more detailed fashion and with reference to Figure 1 which represents the influence of the composition according to the invention on the reduction of itching sensations after 24 days of treatment.

## Detailed description of the invention

### First microbial proliferation inhibitor containing sodium polyacrylate

The first microbial proliferation inhibitor is an aqueous mixture of sodium polyacrylate, polyol compounds, namely glycerine and caprylyl glycol, and at least one glycol compound which is ethoxydiglycol or PEG-8.

Examples of suitable first microbial proliferation inhibitors are sodium polyacrylate / glycerin / ethoxydiglycol / caprylyl glycol / aqua and sodium polyacrylate / glycerine / PEG-8 / caprylyl glycol / aqua sold by the Sederma company under the denominations Osmocide®, Osmocide 2® and Osmocide 3®.

The concentrations of these commercially available materials containing sodium polyacrylate can generally vary from 5 to 25% by weight, the preferred concentrations are usually situated between 10 to 20% by weight relative to the total weight of the composition.

### Second microbial proliferation inhibitor comprising an alkyl glycerine ethers and/or alkyl esters thereof

Suitable alkyl glycerine ethers and alkyl esters thereof are octoxyglycerine (Sensiva SC50®, Phagogene) octoxyglyceryl behenate, octoxyglyceryl palmitate, batyl alcohol (Nikkol Batyl Alcohol 100®, Nikko, Nikkol Batyl Alcohol EX®, Nikko), batyl isostearate (Nikkol GM-181S®, Nikko) and batyl sterate (Nikkol GM-18S®, Nikko).

The concentration of the alkylglycerine ether and/or an alkyl ester thereof generally varies between 0.1 and 2% by weight relative to the weight of the total composition, the preferred concentrations being situated between 0.25 and 1.25% by weight.

Optionally, polysaccharide gums can be used as gelling agents in the the compositions according to the present invention. Suitable polysaccharide gums include:

- the derivatives of modified cellulose, carboxymethyl cellulose (Blanose 7H 3SF®, Aqualon), carboxymethyl hydroxyethyl cellulose (Aqualon), hydroxypropyl cellulose (Klucel H®, Klucel M®, Klucel E®,Aqualon), hydroxyethyl cellulose (Natrosol 250 H®, Natrosol 250 HR®, Natrosol 250 MR®, Aqualon), cetyl hydroxyethyl cellulose (Natrosol 330 CS®, Aqualon), hydroxypropyl methyl cellulose (Methocel E®, Methocel F®, Methocel J®, Methocel K®, Dow Chemical).
- the derivatives of carrageenans, copolymer of sulphoesters of galactose and 3-6-anhydrogalactose (Genugel RLV®, Aqualon), the derivatives of alginates, glyceryl alginate (Karajel®, Chimilux), sodium alginate (Manucol®, SPCI),
- The derivatives of guar, guar gum (Jaguar C®, Rhône-Poulenc), hydroxypropyl guar (Jaguar HP-8®, Jaguar HP-60®, Jaguar HP-79®, Jaguar HP-120® and Jaguar HP-200®, Rhône-Poulenc) and hydroxypropyl guar hydroxypropyltrimonium chloride (Jaguar C-162®, Rhône-Poulenc),

and/or polysaccharide gums obtained by the fermentation of bacteria such as:

- xanthan gums originating from the fermentation of carbohydrates by *Xanthomonas campestris* (Rhône-Poulenc)
- sclerotium gums originating from the fermentation of carbohydrates by *Sclerotium rolfssii* (Amigel®, Alban Muller)
- gellan gums originating from the fermentation of carbohydrates by *Pseudomonas elodea* (Kelcogel®, Kelco).

The concentration of gelling agents, preferably xanthan gum, sclerotium gum and cetyl hydroxyethylcellulose generally varies between 0.05 and 5% by weight relative to the total weight of the composition, the preferred concentrations are situated between 0.1 and 2.5% by weight.

**Immunomodulating molecules**

Several immunomodulating molecules can be used to form the compositions according to the invention. The selection between these molecules can be easily carried out by a person skilled in the art. There can be mentioned amongst others synthetic peptides, biotechnological macromolecules, glycyrrhetinic acid and its stearic ester (Grhetinol-O® and SGS®, Maruzen), plant extracts and extracts from fish eggs such as salmon teleosts (Gamma Nucleotides Marins ®, Seporga).

In a preferred fashion, it is desirable to use immunomodulators which have a minimal number of amino acids situated around 3 or more, preferably around 3 to 8, and which confer on the molecule its immunomodulating properties. This type of peptide chain is coupled with an alkyl chain, usually lipidic, the function of which is to stabilise the molecule and to prevent rapid enzymatic degradation of the peptide chain. In particular, it is desirable to use immunomodulators which do not have antimicrobial properties. In particular, the use of lipoamino acids or antibacterial lipids as described in the Patent FR 2 734 158 is undesirable within the context of the present invention.

Among the preferred synthetic peptides with the context of the present invention, there can be mentioned:

- the alkyl peptides (such as Modulene®, Seporga, in which the peptide sequence is biomimetic of the human melanotropin terminal fraction). The linear or branched alkyl chain contains between 5 and 22, in particular between 8 and 17 carbon atoms.
- melanin activating peptide (MAP®, Seporga), the peptide sequence is derived from human melanotropin.
- epidermal thymosin factor (ETF®, Seporga), the peptide sequence is derived from thymuline.
- cytokin inhibiting factor (Melipren®, Seporga).

Among the biotechnological macromolecules preferred within the context of the present invention, there can be mentioned:

- glycoproteins of yeasts (Immucell®, Pentapharm)
- glycoproteins of the walls of *Enterobacteriae hafnia*
- glycoproteins of the walls of *Klebsiella pneumoniae*
- betaglucan and its copolymers (Polyglucadyne®, Brooks)
- polysaccharides of yeasts such as glucopyranose (Irialin®, Iris, Drieline®, Alban Muller and Glucanne®, Sederma)
- microbiological ferment of *Corynebacterium granulosum* (Langherine®, Sederma).

Among the plant extracts preferred within the context of the present invention, there can be mentioned:

- tropical plant extracts such as Cola, Mate and Guarana (Quench T®, Pentapharm)
- extract of the leaves of Perilla frutescens (Shiso®, Jan Dekker)
- extract of Harpagophyton (Flachmann)
- extract of Coralline (Coralline®, Codif/ADF)
- extract of the root of Echinacea (Echinacea®, Indena).

The concentration of active immunomodulator generally varies between 0.5 and 10% by weight relative to the total weight of the composition, the preferred concentrations being situated between 1 and 5% by weight.

The dermatological and/or cosmetic compositions of the present invention may in addition contain the standard cosmetic and pharmaceutical (dermatological) excipients used such as mineral and vegetable oils, silicon fluids, fatty acids and alcohols, waxes, hydrating agents, vitamins, sequestering agents or any other ingredient usually used in beauty care and dermatology.

The above-mentioned compositions according to the invention can be incorporated into an oil-in-water emulsion, or a water-in-oil emulsion, a water-in-silicone fluid emulsion or a water-in-oil-in-water multiple emulsion. Emulsions contain an oil phase an aqueous phase and an emulsifier to form and stabilise the emulsion. Alternatively the compositions of the present invention may be incorporated into a gel or a pseudo-emulsion (dispersion of two non-miscible phases using gelling agents).

The oil phase of emulsions according to the present invention can contain for example:

1) hydrocarbon oils such as paraffin or mineral oils such as isohexadecane and isododecane;

2) natural oils such as sunflower oil, onager oil, jojoba oil, hydrogenated castor oil, avocado oil, hydrogenated palm oil;

3) natural triglycerides such as caprylic/capric triglyceride, caprylic/capric linoleic triglyceride, caprylic/capric succinic triglyceride;

4) silicon fluids such as cyclomethicone, dimethicone and dimethiconol;

5) fatty alcohols such as stearic alcohol, cetyl alcohol, hexadecylic alcohol;

6) fatty acids such as stearic acid, palmitic acid;

7) esters of fatty acids such as dioctyl succinate, glyceryl dioleate, myristyl myristate and isopropyl myristate;

8) waxes such as beeswax, paraffin, carnauba wax, ozokerite;

9) lanolin and its derivatives (oil, alcohol, waxes);

10) their mixtures.

The oil phase preferably comprises approximately 5 to 30% and preferably 10 to 20% by weight of the composition.

The emulsifiers which can be used in the emulsions according to the present invention can be selected from the emulsifiers known in the prior art as being suitable for use in the types of emulsions listed above. Suitable emulsifiers include:-

1) The sucroesters such as saccharose cocoate and saccharose distearate;

2) The esters of sorbitan and the esters of ethoxylated sorbitan such as sorbitan stearate or polysorbate;

3) The esters of glycerols, the esters of ethoxylated glycerols and polyglycerols such as glyceryl oleate or PEG-20 glyceryl stearate or polyglyceryl-10-stearate;

4) The ethoxylated fatty alcohols such as ceteth-12 or steareth-6;

5) The sesquioleates such as sorbitan sesquioleate;

6) The emulsifiers based on silicon fluid such as silicon polyols;

7) The ethoxylated sterols of soya; or a mixture of one or more of the emulsifiers mentioned above.

The quantity of emulsifiers optionally present in the oil-in-water or the water-in-oil compositions of the present invention is, preferably, from 0.5 to 15% by weight of the composition. The quantity of emulsifiers present in the water-in-oil-in-water multiple emulsion is, preferably approximately 7 to 20% by weight of the composition.

The oil phase of pseudoemulsions according to the present invention may comprise any of the materials listed above as the oil phase of emulsions.

The gels or pseudo-emulsions can be prepared by using one or a combination of several gelling agents selected from the gelling agents acceptable in cosmetic or dermatological products. These gelling agents can also, if desired be incorporated into the emulsions of the present invention. Examples of suitable gelling agents include:-

1) polysaccharide gums such as the derivatives of modified celluloses, derivatives of carraghenans, derivatives of alginates and derivatives of guar;

2) polysaccharide gums obtained from the fermentation of bacteria such as Xanthan gum, Sclerotium gum and Gellan gum;

3) acrylic polymers such as carbomers, polyacrylates, polymetacrylates and their derivatives;

4) quaternized polymers;

5) PVP's and polymers derived from PVP; and

6) mixtures thereof.

In the preferred pseudo-emulsion or in gelled emulsions according to the present invention, the gelling agent or combination of gelling agents represents approximately 0.1 to 30% and preferably approximately 0.25 to 25% by weight of the composition.

In addition, the compositions according to the present invention may contain one or more other compounds which will be known by persons skilled in the art, for example:

1) electrolytes for the stabilization of the water-in-oil emulsion such as sodium chloride or magnesium sulphate, preferably in a quantity ranging from 0.2 to 4% by weight of the composition;

2) moistening agents such as glycerine or propylene glycol or PEG or sorbitol, preferably in a quantity ranging from 1 to 10% by weight of the composition;

3) sequestering agents such as tetrasodium EDTA, preferably in a quantity ranging from 0.01 to 0.5% by weight of the composition;

4) softeners such as the ethers of fatty acids or the esters of fatty acids, preferably in a quantity ranging from 0.5 to 10% by weight of the composition;

5) hydrating agents such as hyaluronic acid, NaPCA, preferably in a quantity ranging from approximately 0.01 to 5% by weight of the composition;

6) film forming agents to facilitate spreading over the surface of the skin, such as the hydorolysates of the proteins of oats, wheat, collagen and almond, preferably in a quantity ranging from approximately 0.1 to 5% by weight of the composition;

7) insoluble pigments such as titanium oxide, rutile titanium oxide, octahedrite titanium oxide, pyrogenic titanium oxide, micronized titanium oxide, titanium oxide surface treated with silicons or with amino acids, or with lecithins, or with metallic stearates, iron oxide, iron oxide surface treated with silicons or with amino acids, or with lecithin, or with metallic stearates, zinc oxide, micronized zinc oxide, mica covered with titanium oxide, preferably in a quantity ranging from approximately 0.5 to 5% by weight of the composition;

8) mixtures thereof

In all that follows and in all that has gone before, the percentages are given by weight except where indicated to the contrary.

Other characteristics and advantages of the invention will become apparent in the examples which follow and are given purely by way of illustration and are in no way limitative. In the Examples that follow the first microbial proliferation inhibitor is an aqueous mixture of sodium polyacrylate (about 0.65%), glycerine (about 52%), caprylyl glycol (about 6%) and ethoxydiglycol (about 20%) sold under the trade name Osmocide by Sederma. The alkyl peptide/dextran component is an alkyl peptide supplied by Seporga under the trade name Modulene

**Example 1:** Day cream (oil-in-water pseudo-emulsion)

Table 1:

| Composition of oil phase | |
| --- | --- |
| **INCI Name** | **Concentration (% weight)** |
| Beeswax | 2 % |
| Isohexadecane | 4 % |
| Dioctyl Succinate | 2 % |
| Isododecane | 3.5 % |
| Glyceryl Dioleate | 3 % |
| Cetyl Alcohol | 2 % |
| Stearic acid | 1 % |

Table 2 :

| Composition of water phase | |
| --- | --- |
| **INCI Name** | **Concentration (% weight)** |
| Sclerotium Gum | 0.35 % |
| Xanthan Gum | 0.35 % |
| Cetyl Hydroxyethylcellulose | 0.35 % |
| Glycerin | 5 % |
| Octoxyglycerin | 1 % |
| Nylon-12 | 2 % |
| First microbial proliferation inhibitor | 15 % |
| Alkyl peptide/Dextran | 3% |
| Aqua | to 100% |

The gelling agents (sclerotium gum, xanthan gum and cetyl hydroxyethylcellulose) are heated to 40°C in 30 ml of water under slow mechanical agitation in order to obtain a translucent gel. Moreover, the other half of the aqueous phase containing the glycerine as well as the oil phase are heated to 80°C.

The aqueous phases and the oil phase were then mixed at 80°C under vigorous agitation. The temperature is maintained at 80°C for 15 minutes and the emulsion is cooled down to 60°C. The first microbial proliferation inhibitor is introduced into the emulsion at this temperature. The other ingredients: octoxyglycerin, alkyl peptide and nylon-12 are added at 30°C. The emulsification is continued until the cream is completely homogeneous.

**Comparative Example 2 :** Day cream (oil-in-water pseudo-emulsion)

Table 3

| Composition of oil phase | |
| --- | --- |
| **INCI Name** | **Concentration (% weight)** |
| Beeswax | 2 % |
| Isohexadecane | 4 % |
| Dioctyl Succinate | 2 % |
| Isododecane | 3.5 % |
| Glyceryl Dioleate | 3 % |
| Cetyl Alcohol | 2 % |
| Stearic acid | 1 % |

Table 4 :

| Composition of aqueous phase | |
| --- | --- |
| **INCI Name** | **Concentration (% weight)** |
| Sclerotium Gum | 0.35 % |
| Xanthan Gum | 0.35 % |
| Cetyl Hydroxyethylcellulose | 0.35 % |
| Glycerin | 5 % |
| Octoxyglycerin | 1 % |
| Nylon-12 | 2 % |
| Alkyl Peptide/Dextran | 3 % |
| Aqua | to 100 % |

The same production process is used as in the preparation of Example 1.

**Example 3 :** Day cream (oil-in-water emulsion containing a liquid crystal phase)

Table 5 :

| Composition of oil phase | |
| --- | --- |
| **INCI Name** | **Concentration (% weight)** |
| Cyclomethicone | 5 % |
| Dioctyl succinate | 3 % |
| Polyacrylamide/C 13-14 Isoparaffin/Laureth-7 | 3 % |
| Palmitic acid | 1 % |
| C12-16 Alcohols | 1 % |

Table 6 :

| Composition of aqueous phase | |
|---|---|
| **INCI Name** | **Concentration (% weight)** |
| Sclerotium Gum | 0.3 % |
| Hydrogenated lecithin | 2 % |
| Glycerin | 5 % |
| Oxtoxyglycerin | 1 % |
| Nylon-12 | 2 % |
| First microbial proliferation inhibitor | 15 % |
| Alkyl peptide/Dextran | 3% |
| Aqua | to 100 % |

The hydrogenated lecithin, 2 % glycerine and 30 ml of water are heated to 70°C under slow mechanical agitation for 30 minutes until hydration of the lecithin is complete. Moreover, the other half of the aqueous phase containing the glycerine at 3 % is used to form the Sclerotium gum gel. In order to do this, the gum and the glycerine are heated at 70°C under slow mechanical agitation.

Before emulsification, the aqueous phases are mixed at 70°C under slow agitation until a homogeneous phase is obtained. The emulsification is carried out by introducing the oil phase, which is also heated to 70°C, into the aqueous phase under vigorous agitation. The emulsion is cooled down under moderate agitation. The same production process as in Example 1 is used to introduce the first microbial proliferation inhibitor, octoxyglycerin, alkyl peptide and nylon-12.

**Example 4 :** Body milk (oil-in-water emulsion)

Table 7 :

| Composition of oil phase | |
|---|---|
| **INCI Name** | **Concentration (% weight)** |
| Isohexadecane | 8 % |
| Mineral oil | 5 % |
| Phenyl dimethicone | 0.5 % |
| Cetyl alcohol | 0.5 % |
| Ceteth-20 | 2 % |
| Sorbitan stearate | 2 % |

Table 8 :

| Composition of aqueous phase | |
|---|---|
| **INCI Name** | **Concentration (% weight)** |
| Sodium carbomer | 0.35 % |
| Glycerin | 5 % |
| Octoxyglycerin | 1 % |
| First microbial proliferation inhibitor | 15 % |
| Alkyl peptide/Dextran | 3% |
| Aqua | to 100 % |

The same production process is used as in the preparation of Example 1.

**Example 5 :** Body cream (water-in-oil emulsion)

Table 9 :

| Composition of oil phase | |
| --- | --- |
| **INCI Name** | **Concentration (% weight)** |
| Isohexadecane | 6 % |
| PEG-30 Dipolyhydroxystearate | 1 % |
| Cyclomethicone/PPG-15 Stearyl Ether | 3 % |

Table 10 :

| Composition of aqueous phase | |
| --- | --- |
| **INCI Name** | **Concentration (% weight)** |
| Magnesium sulphate | 0.8 % |
| Glycerin | 5 % |
| Octoxyglycerin | 1 % |
| First microbial proliferation inhibitor | 15 % |
| Alkyl peptide/Dextran | 3% |
| Aqua | to 100 % |

The same production process is used as in the preparation of Example 1.

**Example 6 :** Eye contour soothing gel

Table 11:

| Composition of aqueous gel | |
| --- | --- |
| **INCI name** | **Concentration (% weight)** |
| Carbomer 980 | 0.50 % |
| Xanthan Gum | 0.20 % |
| PEG-8 | 4 % |
| Octoxyglycerin | 1 % |
| First microbial proliferation inhibitor | 15 % |
| Alkyl peptide/Dextran | 3 % |
| Hafnia extract | 0.03 % |
| Sodium Hyluronate | 0.12 % |
| Methylsilanol Aspartate Hydroxyprolinate | 5 % |
| Tocopheryl Acetate | 0.05 % |
| Tetrasodium EDTA | 0.05 % |
| Sodium hydroxide | 0.15 % |
| Aqua | to 100 % |

The gelling agents (Carbomer 980 and xanthan gum) are heated at 60°C in 30 ml of water under slow mechanical agitation in order to obtain a transparent gel (Phase A).

Moreover, the sodium hyaluronate is dissolved at 40°C in 20 ml of water under magnetic stirring (Phase B).

The remainder of the aqueous phase is used to dissolve (at ambient temperature) the PEG-8, tetrasodium EDTA, tocopheryl acetate, methylsilanol aspartate hydroxyprolinate, Hafnia extract, alkyl peptide and octoxyglycerin (Phase C).

In order to obtain the final product, Phase A, Phase B and the first microbial proliferation inhibitor are mixed together under moderate mechanical agitation.

When the temperature of the gel reaches ambient temperature, Phase C is added. The pH is adjusted with sodium hydroxide.

**Example 7 :** Day cream (water-in-oil-in water emulsion)

Table 12 :

| Composition of primary emulsion | |
|---|---|
| **INCI Name** | **Concentration (% weight)** |
| Oil phase | |
| Glyceryl Isostearate | 2 % |
| PEG-7 Hydrogenated castor oil | 0.2 % |
| Mineral oil/Quaternium 18 hectorite/Propylene Carbonate | 0.2 % |
| Caprylic/Capric Triglyceride | 6.4 % |
| Cyclomethicone | 1.6 % |
| Internal aqueous phase | |
| Glycerin | 0.8 % |
| Magnesium Sulphate | 0.28 % |
| Aqua | to 40 % |

Table 13 :

| Composition of the external aqueous phase | |
|---|---|
| **INCI Name** | **Concentration (% weight)** |
| Carbomer 980 | 0.10 % |
| Acrylates/C 10-30/ Alkyl Acrylate Crosspolymer | 0.60 % |
| Octoxyglycerin | 1 % |
| First microbial proliferation inhibitor | 10 % |
| Alkyl peptide/Dextran | 3 % |
| Tetrasodium EDTA | 0.05 % |
| Sodium hydroxide | 0.15 % |
| Aqua | to 60 % |

The multiple emulsion is prepared according to a two-stage production process.

The first stage consists of producing an oily continuous phase primary emulsion. This is carried out by introducing the internal aqueous phase, heated to 80°C, into the oil phase which is also heated to 80°C under vigorous agitation. Agitation is maintained until ambient temperature is achieved.

The second stage comprises the incorporation of the external aqueous phase containing the gelling agents and the active ingredients into the primary emulsion under weak agitation at ambient temperature. Agitation is maintained until the formation of the multiple emulsion. For the preparation of the external aqueous phase the same production process as in Example 1 is used. At the end of emulsification the pH is adjusted using sodium hydroxide.

**Example 8 :** Sun cream (water-in-oil emulsion)

Table 14 :

| Composition of oil phase | |
|---|---|
| **INCI Name** | **Concentration (% weight)** |
| Cyclomethicone/Dimethicone Copolyol | 6 % |
| PEG-7 Hydrogenated Castor oil | 2 % |
| Cyclomethicone | 2 % |
| Isocetyl stearate | 3 % |
| PEG-45 Dodecyl Glycol Copolymer | 2 % |
| PPG-3 Myristyl Ether | 2 % |
| Hydrogenated Polyisobutene/Titanium Dioxide | 27 % |
| C12-15 Alcohols Benzoate | 2 % |
| Tocepherol | 0.05 % |

Table 15 :

| Composition of the aqueous phase | |
|---|---|
| **INCI Name** | **Concentration (% weight)** |
| Magnesium sulphate | 0.25 % |
| Tetrasodium EDTA | 0.05 % |
| Hafnia extract | 0.02 % |
| PEG-8 | 4 % |
| Octoxyglycerin | 1 % |
| First microbial proliferation inhibitor | 15 % |
| Alkyl peptide/Dextran | 3 % |
| Aqua | to 100 % |

The same production process is used as in the preparation of Example 1.

**Inhibition of microbial proliferation of the combination of the first and second microbial proliferation inhibitors.**

The microbial proliferation inhibitory activity (bacteriostatic and/or fungistatic) of a basic material can be determined by measuring its minimal inhibitory concentration (MIC). This corresponds to the smallest quantity of basic material required to completely inhibit the growth of bacteria or of the yeast tested.

Method for determining the minimal inhibitory concentration (MIC) :

The bacteria of a pure strain of *Staphylococcus epidermidis (Cocci Gram +) IP 8155T* are removed and inoculated in a trypcase-soya liquid medium in order to revivify them for the preparation of mother solutions. The inoculum is produced in such a fashion as to obtain $10^6$ bacteria/ml after incubation in an aerobic atmosphere for 24 hours at 37C. The quantity of bacteria in the suspension is determined by spectrophotometry.

For the determination of the microbiostatic activity, the Mueller Hinton gelose nutrient medium is melted and distributed into Petri dishes.

The samples of basic material tested are prepared extemporaneously. Depending on the basic materials, a successive dilution range is prepared from the mother solution. The necessary quantities are removed from these dilutions and are poured onto the gelose.

Counting of the inoculum is also carried out by the method of decimal dilutions and the necessary quantities of are then deposited on the gelose containing the basic material tested. The Petri dishes are incubated at 37°C for 24 hours.

After incubation for 24 hours, the Petri dishes are examined to determine the concentration of basic material for which there is no culture visible to the naked eye. The lowest concentration at which there was no culture visible is the MIC.

Table 16 :

| Results of the MIC of the first and second microbial proliferation inhibitors | | |
| --- | --- | --- |
| **Basic material tested** | **Concentration %** | **Activity** |
| **First microbial proliferation inhibitor** | 5 | + |
| | 10 | + |
| | 15 | + |
| | 20 | + |
| | 25.40 | + |
| | **26.26** | - |
| | 30 | - |
| | 35 | - |
| | 40 | - |
| **Octoxyglycerine** | 0.1 | + |
| | 0.5 | + |
| | **1** | - |
| **Combination of first microbial proliferation inhibitor and octoxyglycerine** | **15 + 0.5** | - |

The MIC results show that the first microbial proliferation inhibitor has a power to inhibit microbial proliferation starting from 26.26% and the alkyl glycerine ether has an activity starting from 1%. The use of a combination of these two basic materials below their MIC demonstrates a synergistic action.

The dermatological and cosmetic products of the present invention are exposed to the risk of microbiological contamination during their production, their packaging and during their use. For this reason, the effectiveness of the microbial protection of the product must be ensured vis à vis external contamination with the purpose of obtaining a permanent bacteriological quality.

The **challenge test** allows the resistance of a cosmetic product to microbial development to be estimated. This study which is also called "superinfection test", consists of introducing a determined number of known microorganisms, followed by examinations at given times in order to know whether they can survive and multiply. It is supposed to simulate a contamination which occurs during production, packaging or use of the product. This quantitative and qualitative study also allows the selection, in appropriate quantities, of the most effective and the most suitable preservatives for each cosmetic product tested.

Challenge test procedure

The strains of bacteria, yeasts and moulds used in this study are

- bacteria: *Staphylococcus epidermidis* (Cocci Gram +) IP 8155 T *Escherichia coli* (Bacille Gram -) IP 52167 *Pseudomonas aeruginosa* (Bacille Gram -) IP 5842 *Pseudomonas cepacia* (Bacille Gram -) IP 8024 T
- yeast: *Saccharomyces cerrevisiae* IP 118179 (ATC 2601)
- mould: Aspergillus niger IP 143183 (ATC 16404)

The mother solutions of micro-organisms are prepared as in the MIC method. Briefly, the colonies of bacteria, yeasts and moulds are removed and inoculated in the appropriate nutrient medium (trypcase-soya liquid medium for the bacteria and Sabouraud liquid medium for the yeasts and moulds) in order to revive them. The inoculums are carried out so as to obtain $10^6$ micro-organisms/ml after incubation. Each inoculum tube is placed for 24 hours at 37°C for the bacteria and for 72 hours at 30°C for the yeasts and moulds.

The cosmetic products tested are introduced into sterile flasks and contaminated with mother solutions of the micro-organisms ($T_0$). For each micro-organism a different flask is contaminated and the flasks are stored at ambient temperature. At the end of 7 days ($T'_0$) a second contamination is carried out by repeating the previous operation. The product thus polluted is stored at ambient temperature throughout the entire duration of the challenge test.

At the end of the challenge test each contaminated flask is homogenized and for each flask a dilution of 1/10 is carried out in Eugon LT100 broth. The tubes are placed at 37°C for 24 hours and the quantity of living micro-organism is determined by bioluminescence.

The results of the challenge test confirm the MIC results. The same synergistic action is observed for all the micro-organisms tested when the first and second microbial proliferation inhibitors are present in the compositions. By way of example, the results obtained with the bacteria *Staphylococcus epidermis* are shown in Tables 17 and 18. On the other hand, similar tests carried out with the composition of Example 2 have shown that this composition was immediately contaminated by the strains described previously.

Table 17 :

| Results of the challenge test on the composition according to the invention containing 15% of the first microbial proliferation | |
|---|---|
| Time | Relative light unit |
| $T_1$ | 24100 |
| $T_2$ | 6444 |
| $T_7$ | 65 |
| $T'_1$ | 25399 |
| $T'_2$ | 101 |
| $T'_7$ | 89 |

Table 18 :

| Results of the challenge test on the composition according to the invention containing 15% of the first microbial proliferation inhibitor and 1 % of octoxyglycerine | |
|---|---|
| Time | Relative light unit |
| $T_1$ | 13151 |
| $T_2$ | 4114 |
| $T_7$ | 60 |
| $T'_1$ | 255 |
| $T'_2$ | 86 |
| $T'_7$ | 95 |

Detection of the protective effect of the composition according to the invention for hypersensitive and intolerant skins : Stinging Test

Nowadays, it is necessary to ensure that products for topical application are well tolerated without provoking particular reactions such as stinging when they are applied to sensitive areas of the body. To this end, Frosch and Kligman in 1977 perfected a "Stinging Test" in order to determine the intensity of the reactivity of the skin.

The test consists of applying an aqueous solution of lactic acid at a concentration varying from 2 to 10% and physiological saline solution on the nasogenic folds. In fact, this region is known to be particularly reactive as it has a more permeable corneous layer. Moreover, it is rich in hair follicles and sweat glands which encourage the penetration of products applied to the skin and its sensory nerve network is particularly developed. In man, each hair follicle is associated with a specialized nerve ending which gives it a particular sensitivity to touch and pain.

Stinging is a form of pain which is well tolerated by patients. It develops rapidly after the appropriate stimulation of the sensory nerves. In the Stinging Test, evaluation of the stinging sensation is carried out during the first 30 seconds, at 2 minutes and at 5 minutes, by marking from 0 to 3. The score is established by calculating the difference in the sums of the total scores for each sulcus, between the side treated with lactic acid and that treated with physiological saline solution. This test is redone several times with modified protocols depending on the case.

In order to evaluate the protective effect of the present invention on patients who have sensitive and intolerant skins, the stinging test is used according to the following procedure. The study is carried out on 20 female subjects aged between 25 and 50. The subjects are selected from subjects presenting a positive stinging test and having had no local treatment during the week preceding the study.

To qualify the positive stinging test subjects, a 10% aqueous solution of lactic acid is applied using a cotton bud on the left nasogenic sulci of each subject. The right nasogenic sulci of the subjects are used for the application of a placebo, physiological saline solution. The stinging sensation at 10 seconds, 2.5 minutes and 5 minutes is evaluated.

The quantification of the stinging sensation is determined on a numerical scale ranging from 0 to 3. The corresponding scores 0 to 3 signify:

| 0 = absence of stinging | 1 = slight sensation |
| --- | --- |
| 2 = moderate sensation | 3 = intense sensation |

After this evaluation, an **overall score** for the stinging sensation is calculated for each time measurement.

Overall score = ( Σscores for lactic acid) - (Σscores for physiological saline solution).

In order to evaluate the protective effect of the present invention, the product studied is applied on the left nasogenic sulcus and on the left cheek of each subject morning and night for 24 days and the physiological saline solution on the other side under the same conditions. On the 23rd day, the same procedure as that for the qualification of the subjects is repeated. The results obtained are shown in Figure 1.

The use of the composition according to the invention for 24 days allows a reduction in cutaneous activity of 43% to be detected in subjects having a hypereactive or intolerant skin.

**Claims**

1. Dermatological and/or cosmetic composition containing no active or raw ingredient of preservative type and allowing the appearance of the symptoms of hypersensitivity and cutaneous intolerance to be avoided, said composition being **characterized in that** it contains an effective combination of microbial proliferation inhibitors containing (a) a first microbial proliferation inhibitor which comprises a mixture of sodium polyacrylate, glycerine, caprylyl glycol, aqua, and ethoxydiglycol or PEG-8, and (b) a second microbial proliferation inhibitor which comprises at least one monoalkyl glycerine ether or an ester thereof selected from octoxyglycerine, octoxyglyceryl behenate, octoxyglyceryl palmitate, batyl alcohol, batyl isostearate and batyl stearate.

2. Composition according to claim 1, in which the alkyl glycerine is octoxyglycerine.

3. Composition according to any preceding claim which in addition contains at least one polysaccharide gum.

4. Composition according to claim 3, in which the polysaccharide gum is cetylhydroxyethylcellulose.

5. Composition according to claim 3, in which the polysaccharide gum is obtained by fermentation and is preferably sclerotium gum or xanthan gum.

6. Composition according to any one of the preceding claims, which in addition contains an immunomodulating molecule.

7. Composition according to claim 6, in which the immunomodulating molecule is an alkyl peptide in which the peptide sequence is biomimetic of the human melanotropin terminal fraction and the alkyl moiety is a linear or branched alkyl chain containing between 5 and 22 carbon atoms.

8. Composition according to any one of the preceding claims, in which the combination of the microbial proliferation inhibitors is present in a quantity of approximately 0.25 to 30% by weight of the composition.

9. Composition according to claim 1, in which the mixture of sodium polyacrylate, glycerine, caprylyl glycol, aqua and ethoxydiglycol or PEG-8 is present in a quantity of approximately 10 to 20%, preferably approximately 15% by weight of the total composition.

10. Composition according to claim 8, in which the alkyl glycerine and/or the alkyl ester thereof is present in a quantity of approximately 0.25 to 1.25%, preferably approximately 0.5% by weight of the total composition.

11. Composition according to any one of claims 3 to 5, in which the polysaccharide gum is present in a quantity of approximately 0.05 to 5% by weight of the total composition.

12. Composition according to claim 6 or 7, in which the immunomodulating molecule is present in a quantity of approximately 1 to 5% by weight of the total composition.

13. Composition according to any one of the preceding claims, **characterized in that** the said composition is in the form of a pseudo-emulsion, or an oil-in-water emulsion, or a water-in-oil emulsion, or a water-in-silicon fluid emulsion , or a gel, or a water-in-oil-in-water multiple emulsion.

14. Composition according to claim 13, **characterized in that** the said composition is preferably in the form of a pseudo-emuision or an oii-in-water emulsion.

15. Non-therapeutic local or topical application of the composition according to any one of claims 1 to 7.

16. Use of a composition according to any one of claims 1 to 20 for the preparation of a medicament intended for the treatment of the symptoms of hypersensitivity and cutaneous intolerance, for the treatment of the symptoms of psoriasis, for treatment of the symptoms of atopy, for the treatment of the symptoms of acne, for the treatment of the symptoms of cutaneous ageing, or for the prevention of the symptoms of benign estival light eruption.

**Patentansprüche**

1. Dermatologische und/oder kosmetische Zusammensetzung, die keine aktiven oder rohen Wirkstoffe konservierender Art enthält und es erlaubt, das Auftreten von Symptomen der Hypersensibilität und Unverträglichkeit der Haut zu verhindern, wobei die genannte Zusammensetzung **dadurch gekennzeichnet ist, dass** sie eine wirksame Kombination aus Inhibitoren der Mikrobenausbreitung enthält, die (a) einen ersten Inhibitor der Mikrobenausbreitung enthalten, welcher eine Mischung aus Natriumpolyacrylat, Glycerin, Caprylylglykol, Wasser und Ethoxydiglykol oder PEG-8 umfasst, und die (b) einen zweiten Inhibitor der Mikrobenausbreitung enthalten, welcher mindestens einen Glycerinmonoalkylether oder einen Ester desselben umfasst, ausgewählt aus Oktoxyglycerin, Oktoxyglyceryl Behenat, Oktoxyglyceryl Palmitat, Batylalkohol, Batyl Isostearat und Batyl Stearat.

2. Zusammensetzung nach Anspruch 1, wobei das Alkylglycerin ein Oktoxyglycerin ist.

3. Zusammensetzung nach einem der vorangehenden Ansprüche, welche zudem mindestens ein Polysaccharidgummi enthält.

4. Zusammensetzung nach Anspruch 3, wobei das Polysaccharidgummi Cetylhydroxyethylcelullose ist.

5. Zusammensetzung nach Anspruch 3, wobei das Polysaccharidgummi durch Fermentation erhalten wird und vorzugsweise Sclerotiumgummi oder Xanthikgummi ist.

6. Zusammensetzung nach einem der vorangehenden Ansprüche, welche zudem ein immunmodulierendes Molekül enthält.

7. Zusammensetzung nach Anspruch 6, wobei das immunmodulierende Molekül ein Alkylpeptid ist, in welchem die Peptidsequenz in Bezug auf die Endfraktion des humanen Melantropins biomimetisch ist und die Alkylfraktion eine geradkettige oder verzweigte, 5 bis 22 Kohlenstoffatome enthaltende Alkylkette ist.

8. Zusammensetzung nach einem der vorangehenden Ansprüche, wobei die Kombination der Inhibitoren der Mikrobenausbreitung in einer Menge von ca. 0,25 bis 30 Gew.-% der Zusammensetzung vorhanden ist.

9. Zusammensetzung nach Anspruch 1, wobei die Mischung aus Natriumpolyacrylat, Glycerin, Caprylylglykol, Wasser und Ethoxydiglykol oder PEG-8 in einer Menge von ca. 10 bis 20 Gew.-%, vorzugsweise von ca. 15 Gew.-% der gesamten Zusammensetzung vorhanden ist.

10. Zusammensetzung nach Anspruch 8, wobei das Alkylglycerin und/oder der Alkylester derselben in einer Menge von ca. 0,25 bis 1,25 Gew.-%, vorzugsweise von ca. 0,5 Gew.-% der gesamten Zusammensetzung vorhanden ist.

**11.** Zusammensetzung nach einem der Ansprüche 3 bis 5, wobei das Polysaccharidgummi in einer Menge von ca. 0,05 bis 5 Gew.-% der gesamten Zusammensetzung vorhanden ist.

**12.** Zusammensetzung nach Anspruch 6 oder 7, wobei das immunmodulierende Molekül in einer Menge von ca. 1 bis 5 Gew.-% der gesamten Zusammensetzung vorhanden ist.

**13.** Zusammensetzung nach einem der vorangehenden Ansprüche, **dadurch gekennzeichnet, dass** die genannte Zusammensetzung in Form einer Pseudoemulsion oder einer Öl-in-Wasser-Emulsion oder einer Wasser-in-Öl-Emulsion oder einer flüssigen Wasser-in-Silikon-Emulsion oder eines Gels oder einer mehrfachen Wasser-in-Öl-in-Wasser-Emulsion vorhanden ist.

**14.** Zusammensetzung nach Anspruch 13, **dadurch gekennzeichnet, dass** die genannte Zusammensetzung vorzugsweise in Form einer Pseudoemulsion oder einer Öl-in-Wasser-Emulsion vorhanden ist.

**15.** Lokale oder örtliche nicht therapeutische Anwendung der Zusammensetzung nach einem der Ansprüche 1 bis 7.

**16.** Verwendung einer Zusammensetzung nach einem der Ansprüche 1 bis 20 zur Herstellung eines Medikaments für die Behandlung der Symptome der Hypersensibilität oder Unverträglichkeit der Haut, für die Behandlung der Symptome der Psoriasis, für die Behandlung der Symptome der Atopie, für die Behandlung der Symptome der Akne, für die Behandlung der Symptome der Hautalterung oder für die Vorbeugung der Symptome des gutartigen leichten Sommerausschlags.

## Revendications

**1.** Composition dermatologique et/ou cosmétique ne contenant aucun ingrédient actif ou matière première de type conservateur et permettant d'éviter l'apparition de symptômes d'hypersensibilité et d'intolérance cutanée, ladite composition étant **caractérisée en ce qu'**elle contient une combinaison efficace d'inhibiteurs de prolifération microbienne contenant (a) un premier inhibiteur de prolifération microbienne qui comprend un mélange de polyacrylate de sodium, glycérine, caprylyl glycol, aqua, et éthoxydiglycol ou PEG-8, et (b) un second inhibiteur de prolifération microbienne comprenant au moins un éther de monoalkyl glycérine ou un ester de celui-ci choisi parmi l'octoxyglycérine, l'octoxyglycéryl béhénate, l'octoxyglycéryl palmitate, l'alcool batylique, l'isostéarate batylique et le stéarate batylique.

**2.** Composition selon la revendication 1, dans laquelle l'alkyl glycérine est l'octoxyglycérine.

**3.** Composition selon n'importe quelle revendication précédente contenant en outre au moins une gomme polysaccharidique.

**4.** Composition selon la revendication 3, dans laquelle la gomme polysaccharidique est la cétyl hydroxyéthylcellulose.

**5.** Composition selon la revendication 3, dans laquelle la gomme polysaccharidique est obtenue par fermentation et est de préférence la gomme de sclérote ou la gomme xanthane.

**6.** Composition selon l'une quelconque des revendications précédentes, qui contient en outre une molécule immunorégulatrice.

**7.** Composition selon la revendication 6, dans laquelle la molécule immunorégulatrice est un alkyl peptide dans lequel la séquence peptidique est biomimétique de la fraction terminale de la mélanotropine humaine et la fraction alkyle est une chaîne alkyle linéaire ou ramifiée contenant entre 5 et 22 atomes de carbone.

**8.** Composition selon l'une quelconque des revendications précédentes, dans laquelle la combinaison des inhibiteurs de prolifération microbienne est présente en une quantité d'environ 0,25 à 30% en poids de la composition.

**9.** Composition selon la revendication 1, dans laquelle le mélange de polyacrylate de sodium, de glycérine, de caprylyl glycol, d'aqua et d'éthoxydiglycol ou de PEG-8 est présent en une quantité d'environ 10 à 20%, de préférence environ 15% en poids de la composition totale.

**10.** Composition selon la revendication 8, dans laquelle l'alkyl glycérine.et/ou l'alkyl ester de celle-ci est présent en une quantité d'environ 0,25 à 1,25%, de préférence environ 0,5% en poids de la composition totale.

**11.** Composition selon l'une quelconque des revendications 3 à 5, dans laquelle la gomme polysaccharidique est présente en une quantité d'environ 0,05 à 5% en poids de la composition totale.

**12.** Composition selon la revendication 6 ou 7, dans laquelle la molécule immunorégulatrice est présente en une quantité d'environ 1 à 5% en poids de la composition totale.

**13.** Composition selon l'une quelconque des revendications précédentes, **caractérisée en ce que** ladite composition est sous forme d'une pseudo-émulsion, ou d'une émulsion huile dans eau, ou d'une émulsion eau dans huile, ou d'une émulsion eau dans huile de silicone, ou d'un gel, ou d'une émulsion multiple eau dans huile dans eau.

**14.** Composition selon la revendication 13, **caractérisée en ce que** ladite composition est de préférence sous forme d'une pseudo-émulsion ou d'une émulsion huile dans eau.

**15.** Application locale ou topique non thérapeutique de la composition selon l'une quelconque des revendications 1 à 7.

**16.** Utilisation d'une composition selon l'une quelconque des revendications 1 à 20 pour la préparation d'un médicament destiné au traitement des symptômes d'hypersensibilité et d'intolérance cutanée, au traitement des symptômes du psoriasis, au traitement des symptômes de l'atopie, au traitement des symptômes de l'acné, au traitement des symptômes du vieillissement cutané, ou à la prévention des symptômes de lucite estivale bénigne.

Fig.1

**EP 0 996 418 B2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- FR 2682296
- WO 9705856 A
- EP 0747047 A
- FR 27229050 A
- DE 4140474 A
- US 5621012 A
- WO 9637180 A
- FR 2734158